# EUROPEAN PATENT APPLICATION

(11) **EP 3 351 287 A1**
(43) Date of publication of application: **25.07.2018**
(21) Application number: 16846308.1
(22) Date of filing: 06.09.2016
(51) Int. Cl.: A61M 37/00

(54) **MICRO-NEEDLE**

(30) Priority: 17.09.2015 JP 2015183735
(71) Applicant: AOF Pte. Ltd., Shinagawa-ku, Tokyo 141-0022 (JP)
(72) Inventor: ENOMOTO Hideharu, Tokyo 141-0022 (JP); SHINOHARA Masumi, Tokyo 141-0022 (JP); TERADA Tokinori, Yokohama-shi Kanagawa 224-0014 (JP)
(74) Representative: Dehns
(86) International application number: PCT/JP2016/076112
(87) International publication number: WO 2017/047437

(57) **Abstract**

The purpose of the present invention is to provide a micro-needle having a leading end that can reach the dermis with certainty by penetrating through the epidermis when piercing the skin and that can supply a drug or the like to the dermis or to a region deeper than the dermis.

Accordingly, this micro-needle (10) has a base (1) and a plate-shaped blade (2). The plate-shaped blade (2) and the base (1) are formed in an integrated manner, and the plateshaped blade (2) extends from an end (1A: step section) of the base (1). Alternatively, the present invention is characterized by having a base (111) and a conical leading end part (112) and a star-like polygonal cross-sectional shape part (114, 124) being formed in a region between the base (111) and the conical leading end part (112), and in that the base (111), the conical leading end part (112) and the starlike polygonal cross-sectional shape part (114, 124) are formed in an integrated manner.

## Description

### TECHNICAL FIELD

The present invention relates to a microneedle.

### BACKGROUND ART

In recent years, a microneedle has been rapidly spread as a technique for simply and certainly supplying a drug (medicine), a beauty article, or another substance contributing to the activity of the skin (hereinafter referred to as "drug or the like" which includes medicines) to a skin surface layer.

Herein, in order to supply the drug or the like by the microneedle to activate the skin, and especially the epidermis, it is necessary that during puncture with the microneedle through the skin, a tip of the microneedle certainly penetrates the epidermis and reaches to the dermis so as to supply the drug or the like to the dermis.

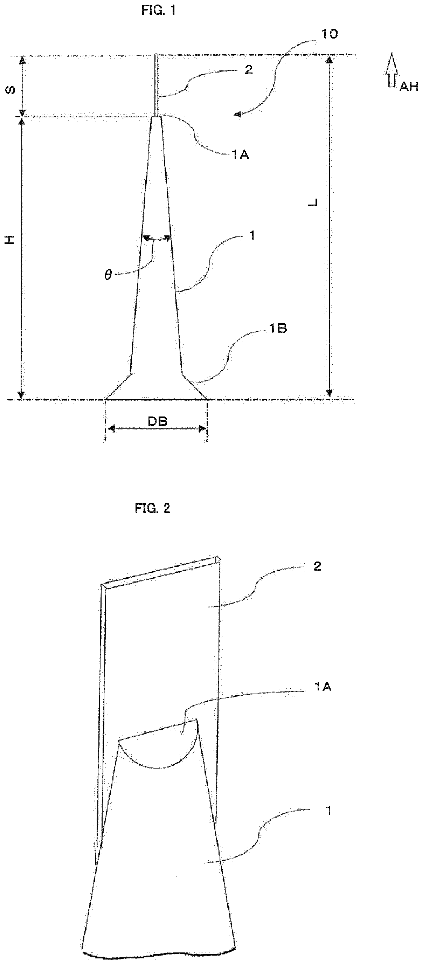

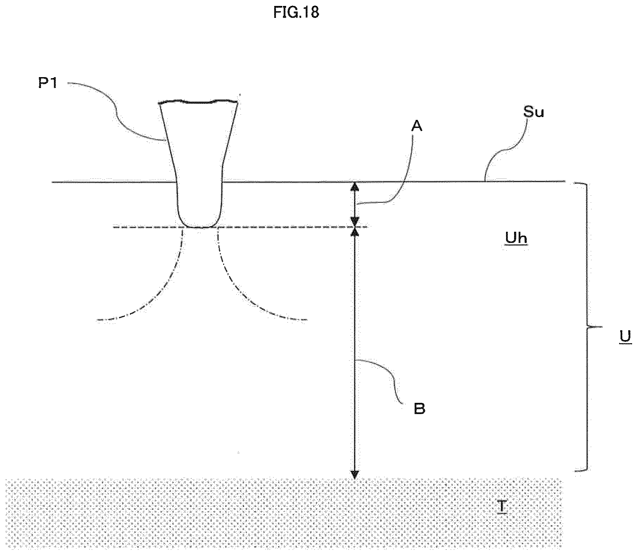

However, as shown in FIG. 18, a microneedle of the prior art represented by a reference symbol P1 is allowed to puncture the skin, for example, by only about 60 µm from the tip of the microneedle (region A) . In a region B on a side of the dermis T (in FIG. 1, a lower region: region being apart from a surface Su), the tissues of the skin are only pressed by the microneedle P1 and the tip of the microneedle P1 is not inserted into the tissues of the skin. Therefore, the prior art microneedle P1 allows the drug or the like to be only supplied to a part of stratum corneum Uh in the epidermis Uh (i.e., the stratum corneum Uh constituting a part of the epidermis U). The prior art microneedle P1 could not supply the drug or the like to the dermis T, and therefore, could not activate the dermis T.

Although there are differences of a part to be punctured with the microneedle and an individual variation, in general, a distance between a surface Su of the skin and the dermis T is, for example, about 800 µm. In the microneedle P1 of the prior art, even if the tip thereof is inserted into the skin, the microneedle P1 must be inserted to the side of the dermis T by about 800 µm. In this case, there is a risk that the prior art microneedle P1 is broken on the way.

As mentioned above, in the prior art microneedle, in a case that the tip of the microneedle is pressed against the skin, the prior art microneedle cannot penetrate the epidermis and cannot reach to the dermis in many cases. Therefore, it is difficult to activate the skin at a desired level.

As another prior art, there is a microneedle having a tip part with a sharp of frustum of cone (see Patent Document 1) . However, although that the tip thereof can pass through the stratum corneum, it is difficult for the tip to penetrate the epidermal due to the same reason as described above.

### PRIOR ART DOCUMENT

### PATENT DOCUMENT

Patent Document 1: Japanese Patent No. 5050130

### SUMMARY OF THE INVENTION

### PROBLEMS TO BE SOLVED BY THE INVENTION

In view of the above-mentioned problems in the prior arts, the present invention has been proposed. An object of the present invention is to provide a microneedle in which a tip certainly penetrates the epidermis and reaches to the dermis during puncture through the skin, to supply a drug or the like to the dermis or a region deeper than the dermis (a region apart from the skin surface).

### SOLUTIONS TO THE PROBLEMS

A microneedle (10) of the present invention comprises a base (1) and a plate-shaped blade (2), wherein the base (1) and the plate-shapedblade (2) are integrally formed and the plate-shaped blade (2) extends from an end part (1A: stair part or shoulder part) of the base (1).

Alternatively, a microneedle (110) of the present invention comprises a base (111) and a conical tip part (112), wherein a part having a star polygonal cross section (114 and 124: the cross-sectional shape is, for example, star pentagon or star hexagon) is formed in a region between the base (111) and the conical tip part (112), and the base (111), the conical tip part (112) and the part having a star polygonal cross section (114 and 124) are integrally formed.

In the present invention, it is preferable that the microneedle (10 and 110) is constructed by a drug or the like, for example, hyaluronic acid (including low molecular weight hyaluronic acid and hyaluronic acid other than the low molecular weight hyaluronic acid), a hair growth promoter, a supernatant of a culture broth in which stem cells are cultured, another material capable of activating the skin (a material for manufacture of microneedles), and insulin. It is preferable that the material is in a liquid form and is capable of being solidified.

The drug or the like as the material for manufacture of microneedles widely includes an anticancer drug, a hair growth promoter, a therapeutic drug for an auditory disorder, a therapeutic drug for allergy (e.g., "allergy to pollen"), a therapeutic drug for empyema, a drug for Alzheimer's disease and other dementia, a drug for a brain disease, a therapeutic drug for laryngitis, a therapeutic drug for a periodontal disease, a drug for regeneration of alveolar bone, a drug for improvement of oral cavity immunity, a therapeutic drug for prevention of sunburn and inflammation caused by sunburn, a therapeutic drug for psoriasis, a therapeutic drug for atopic dermatitis, a therapeutic drug for decubitus (bedsore), a therapeutic drug for other various skin diseases, a drug for regeneration of skin to remove stretch marks, a drug for regeneration of skin after wound and another surgery, a therapeutic drug for a disease caused by Trichophyton, a drug for prevention of an increase in puncture and ED, and other various drugs (e.g. , herbal drug) . In addition, the drug or the like as the material for manufacture of microneedles is intended to include not only a drug for human but also a drug for animals such as dogs, cats, and domestic animals.

The microneedle (10 and 110) of the present invention does not have a space inside thereof (so-called "solid"). However, the microneedle may have a space inside thereof (so-called "hollow").

A method for manufacturing the microneedle (10) of the present invention comprises steps:
for filling a mold (20 and 30) with a liquid material for manufacture of microneedles, saidmold (20 and 30) having a space (CD) having a complementary shape with the base (1) of the microneedle (10) and a space (CB: penetrating groove) having a complementary shape with the plate-shaped blade (2), the space (CB) having a complementary shape with the plate-shaped blade (2) communicating with an external space (CO: hollow part) of the mold (20 and 30) (spaces CB penetrates the mold 20 and 30) ; and
for decompressing the space (CO: the external space or the hollow part) communicating with the space (CB) having a complementary shape.

Also, the mold (20 and 30) used in manufacture of the microneedle (10) of the present invention has the space (CD) having a complementary shape with the base (1) of the microneedle (10) and the space (CB: penetrating groove) having a complementary shape with the plate-shaped blade (2), and the space (CB) having a complementary shape with the plate-shaped blade (2) communicates with the external space (CO) (spaces CB penetrates the mold).

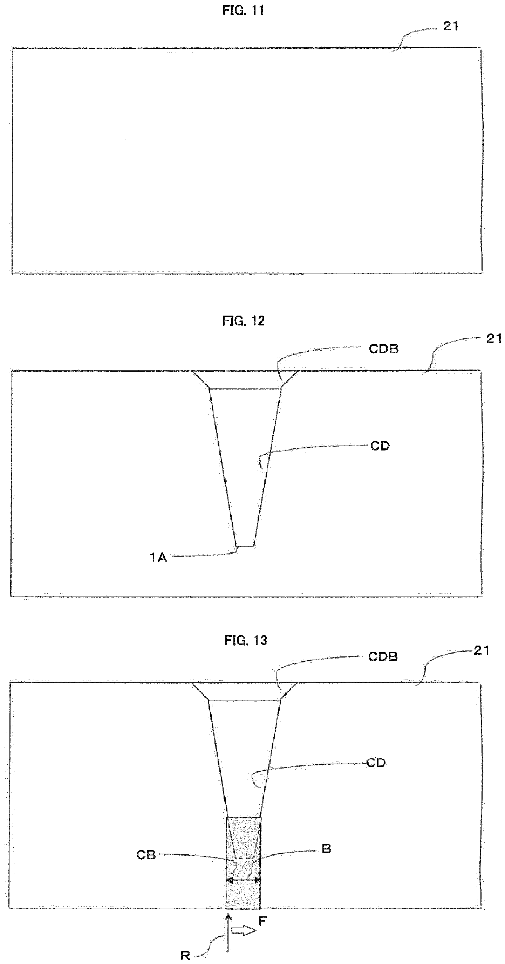

Further, a method for manufacturing the mold (20) used in manufacture of the microneedle (10) of the present invention (shown in Figs. 11 to 13) comprises steps:
for processing a complementary region (CD and CDB) with the base (1) of the microneedle (10); and
for cutting the mold (20) so as to form the space (CB: penetrating groove) having a complementary shape with the plate-shaped blade (2) by irradiating a laser beam from the bottom of the mold (20) so that the laser beam penetrates to the complementary region (CD and CDB) with the base (1) of the microneedle (10) and moving the laser beam by a width (B) of the blade (2) in a horizontal direction (a direction orthogonal to the longitudinal direction of the complementary region CD and CDB with the base 1 of the microneedle 10).

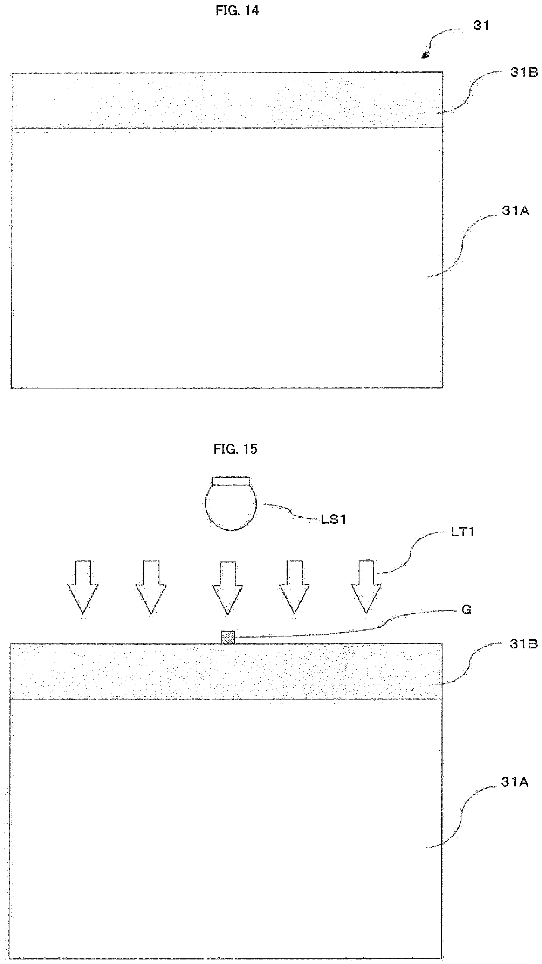

Alternatively, a method for manufacturing the mold (30) used in manufacture of the microneedle (10) of the present invention (Figs. 14 to 17) comprises steps:
for using a workpiece (31) being constructed by a workpiece body part (31A) made of a permeable material (glass or the like) and a photoresist part (31B) formed from a photoresist (photoresist to be cured by irradiation with light),
for placing a shading member (G) having the same size as that of a tip surface of the plate-shaped blade (2) of the microneedle (10) on the photoresist part (31B) and irradiating with light to the photoresist part (31B) from a side on which the shading member (G) is placed (the upper side of the mold 30) ;
for forming a space (CB: penetrating groove) having a complementary shape with the blade (2) by removing the (uncured) photoresist directly below said shading member (G); and
for forming a space (CD and CDB) having a complementary shape with the base (1) by irradiating with light to the workpiece body (31A) from a side of the photoresist part (31B) and by processing the workpiece body (31A) from a side opposite to the light irradiation side (laser processing, for example, processing through irradiation with pulsed laser light).

In the above-mentioned manufacturing method (Figs. 14 to 17), the space CB having a complementary shape with the blade 2 is formed by means of so-called "dry etching" and "wet etching. "

### EFFECTS OF THE INVENTION

By means of the microneedle (10) of the present invention having the above-mentioned constructions, since the plate-shaped blade (2) is integrally formed at an end part of the base (1), in a case that the blade (2) has a small thickness and a sharp shape, the blade (2) allows a skin tissue to be incised. Based on that the skin tissue is incised by the blade (2), the base (1) integrally molded with the blade (2) can also easily be inserted into the skin tissue.

Accordingly, themicroneedle (10) is inserted into the dermis (T), a drug or the like as a material constructing the microneedle (10) is supplied to the dermis (T), and the skin is activated.

Alternatively, according to the microneedle (110) of the present invention, since the part having a star polygonal cross section (114 and 124: the cross-sectional shape is, for example, star pentagon or star hexagon) is formed in a region between the base (111) and the conical tip part (112), a tip (114P) of the part having a star polygonal cross section (114 and 124) allows the skin tissue to be incised, and therefore, a region on a side apart from the conical tip part (112) as compared with the part having a star polygonal cross section (114 and 124) of the microneedle (110) can be easily inserted into the skin tissue, and therefore, the microneedle (110) can be easily inserted into the dermis (T).

As a result, a drug or the like as a material constructing the microneedle (110) is supplied to the dermis (T), and then, the skin is activated.

Herein, in a case that an end part on the side of the conical tip part (112) of the part having a star polygonal cross section (124) constructs a sharp top part (124TP) protruding on the side of the conical tip part (112), not only the tip (124P) of the part having a star polygonal cross section (124) but also the sharp top part (124TP) allow the skin tissue to be incised, and therefore, the region on the side apart from the conical tip part (112) as compared with the part having a star polygonal cross section (124) is easily inserted into the skin tissue furthermore.

Also, the plate-shaped blade (2) is easily to be manufactured by using a mold.

Unlikely in a case that a needle-shaped member is manufactured using a mold, in manufacture of the plate-shaped blade (2) by means of a mold, the tip shape is not formed into a spherical shape or a curved shape, and therefore, the blade (2) allows the skin tissue to be certainly incised and the microneedle (10) can be inserted into the dermis (T).

In a case that the dimension and the strength of the blade (2) are appropriately designed, during incision of the skin tissue and insertion, a bending of the blade (2) can be prevented, as compared with the needle-shaped member.

According to the above-mentioned method for manufacturing the microneedle (10) of the present invention, a penetrating groove is formed in an area being below the region having a complementary shape with the base (2) (an area of the region CB having a complementary shape with the blade 2) in the using mold (20 and 30), the step for communicating a lower side (a side of the region CB having a complementary shape with the blade 2) of the mold (20 and 30) with an environment of reduced pressure is carried out, and then, a material for molding of the microneedle (10) (liquid material) is adsorbed due to a negative pressure, and then, the space (regions CB, CD, and CDB) in the mold (20 and 30) is certainly filled with the material.

Therefore, it is possible to prevent a case that the mold (20 and 30) is not filled to the tip (whole region CB) thereof with the material for molding due to surface tension. Although gas is generated, the mold (20 and 30) is filled to the tip thereof (the whole region CB) with the material for molding.

Further, due to the microneedle (10) of the present invention is manufactured using the mold (20 and 30), a sheet-shaped member in which many microneedles (10) are fixed can be easily manufactured.

By means of connecting the sheet-shaped member to another sheet-shaped member, it is possible to manufacture easily a large area sheet to which many microneedles (10) are fixed. In a case that such a large area sheet is appropriately processed, it is possible to provide a device being suitable for a shape of use part (e.g., a hair part) of individual user, that is, a "made-to-order" device can be provided.

According to the above-mentioned method for manufacturing the mold (20) used in the method for manufacturing the microneedle (10) of the present invention (shown in Figs. 11 to 13), since the region (CD and CDB) being complementary with the base (1) of the microneedle (10) is processed and then the mold (20) is irradiated from the bottom thereof with a laser beam in order to cut the penetrating groove (CB), the base (1) and the blade (2) of the microneedle (10) manufactured using the mold (20) are integrally manufactured, and the base (1) and the blade (2) are not separated.

Further, according to the method for manufacturing the mold (30) used in the method for manufacturing the microneedle (10) of the present invention (shown in Figs. 14 to 17), a part forming the space (CB: penetrating groove) having a complementary shape with the plate-shaped blade (2) in the mold (30: workpiece 31) is made of a photoresist (photoresist part 31B), and is blocked (shaded) against light by the small shading member (G) corresponding to the cross-sectional shape of the space (CB). Thus, a region in which the space CB should be formed in the photoresist part (31B) is not cured, and the photoresist region can be easily removed.

Therefore, in a case that a space (CB: slit SL: penetrating groove) having a complementary shape with the blade (2) cannot be formed by a laser, a space (CB: penetrating groove) having a small thickness (W) can be easily and precisely formed.

Since the thickness of the formed space (CB) is increased toward the side of the base (1), it is convenient to pull the microneedle from the mold.

As mentioned above, by forming the penetrating groove (CB), the entire region of the mold (20 and 30) is filled with the material for molding, by decompressing the lower side of the mold (20 and 30) during molding of the microneedle (10).

Therefore, it is possible to prevent a case that the material for molding cannot be inserted into the end part of the mold (20 and 30) due to the surface tension of the material for molding and generation of gas, and to prevent a case that a microneedle (10) having a predetermined shape cannot be obtained as a result.

### BRIER DESCRIPTION OF THE DRAWINGS

FIG. 1 is an explanatory view illustrating an outline of a microneedle according to a first embodiment of the present invention;
FIG. 2 is a partially enlarged perspective view of the microneedle shown in FIG. 1;
FIG. 3 is a partially enlarged front view of the microneedle shown in FIG. 1;
FIG. 4 is a partially enlarged side view of the microneedle shown in FIG. 1;
FIG. 5 is a partially enlarged perspective view of a microneedle according to a first modification of the first embodiment;
FIG. 6 is a partially enlarged perspective view of a microneedle according to a second modification;
FIG. 7 is a flow chart illustrating a procedure of manufacturing the microneedle according to the first embodiment;
FIG. 8 is an explanatory view illustrating one step in manufacture of the microneedle according to the first embodiment;
FIG. 9 is a cross-sectional view illustrating a mold used in manufacture;
FIG. 10 is a cross-section view at a section indicated by arrows K-K shown in FIG. 9;
FIG. 11 is an explanatory view illustrating a workpiece of a mold before manufacture of the mold in a method for manufacturing a mold for the microneedle according to the first embodiment;
FIG. 12 is an explanatory view illustrating one step in manufacture of the mold for the microneedle according to the first embodiment using the workpiece shown in FIG. 11;
FIG. 13 is an explanatory view illustrating one step in processing a penetrating groove in the mold, which is a step following the step shown in FIG. 13;
FIG. 14 is an explanatory view illustrating a workpiece used in a method for manufacturing a mold, which method is different from the method shown in Figs. 11 to 13;
FIG. 15 is an explanatory view illustrating a step of processing the mold for the microneedle according to the first embodiment using the workpiece shown in FIG. 14;
FIG. 16 is an explanatory view illustrating a step following the step shown in FIG. 15;
FIG. 17 is an explanatory view illustrating a step following the step shown in FIG. 16;
FIG. 18 is a schematic view illustrating an outline of a prior art;
FIG. 19 is an explanatory view illustrating an outline of a microneedle according to a second embodiment of the present invention;
FIG. 20 is a partially enlarged perspective view of the microneedle shown in FIG. 19;
FIG. 21 is a partially enlarged plan view of the microneedle shown in FIG. 19;
FIG. 22 is a partially enlarged front view of the microneedle shown in FIG. 19;
FIG. 23 is a cross-section view at a section indicated by arrows A23 - A23 of the microneedle shown in FIG. 19; and
FIG. 24 is a partially enlarged front view illustrating a main part of a modification of the second embodiment.

### BEST MODE FOR CARRYING OUT THE INVENTION

Hereinafter, embodiments of the present invention will be described with reference to the appended drawings.

With reference to Figs. 1 to 4, a microneedle 10 according to a first embodiment will be explained at first.

It is to be noted that, for easiness of description, Figs. 1 to 4 are not drawn to the same scale, and a ratio of size of members is expressed differently depending on each drawing.

In FIG. 1, a microneedle being entirely represented by a reference symbol 10 has a plate-shaped blade 2 on a tip side (an arrow AH side) and a base 1 which is integrally molded with the plate-shaped blade 2.

The length of the microneedle 10 in a central axis direction is represented by a reference symbol L, the length of the base 1 in the central axis direction is represented by a reference symbol H, and the length of the plate-shaped blade 2 in the central axis direction is represented by a reference symbol S.

As clearly shown in FIG. 2, the base 1 in the first embodiment is formed in a shape of frustum of cone, and a tip angle of the base 1 on a side of the plate-shaped blade 2 is represented by a reference symbol θ.

The diameter of a bottom surface part 1B on a lower side of the base 1 is abruptly increased.

In a case that a large number of microneedles 10 should be attached to one sheet (in a case that an assembly of sheet-shaped members, not shown, should be manufactured), each manufactured microneedle 10 is bonded to the sheet-shaped member coated with an adhesive (not shown) and stored, the bottom surface part 1B is certainly bonded to the sheet-shaped member due to the large diameter of the bottom surface part. Herein, the maximum diameter (the diameter of a lower end part) of the bottom surface part 1B is represented by a reference symbol DB.

It is to be noted that the base bottom surface part 1B (the part in which the diameter is abruptly increased) may be omitted.

In the first embodiment, since a tip of the microneedle 10 is the plate-shaped blade 2, the blade 2 has a sharp shape and can cut the skin tissue to be incised. When the skin tissue is incised by the blade 2, the base 1 integrally molded with the blade 2 can also easily be inserted into the skin tissue.

Since the tip of the microneedle 10 is the plate-shaped blade 2, the microneedle 10 is easily manufactured by means of a "mold." That is, in a case that a needle-shaped member is manufactured by means of a "mold, " a tip shape having a smaller diameter may be formed in a spherical shape or a curved shape. For this reason, it is difficult that the skin tissue is punctured with the needle-shaped member.

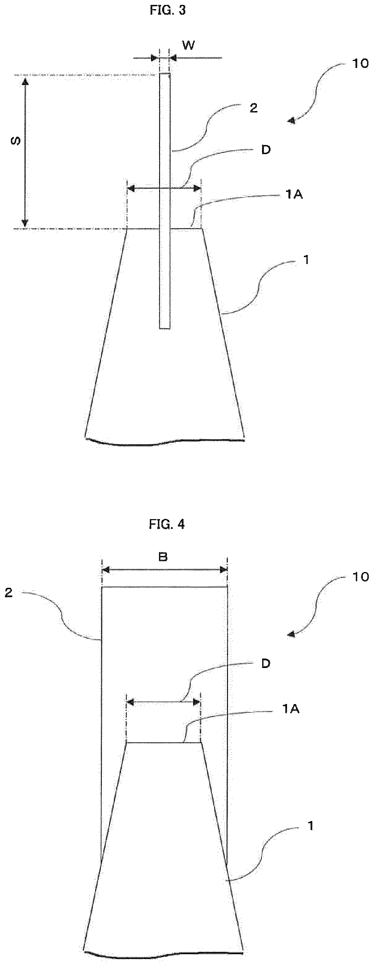

On the other hand, in the plate-shaped blade 2 as shown in Figs. 1 to 4, although a thickness W (FIG. 3) thereof is small, the tip thereof may not be formed in a spherical shape or a curved shape during manufacture by means of the mold. Therefore, the plate-shaped blade 2 can cut the skin tissue to be incised and the tip can be inserted into the skin tissue.

Further, since the dimensions (thickness W, width B, length S, and Figs. 3 and 4) and the strength of the blade are appropriately design in consideration of a part to be punctured with the microneedle 10 and the like, bending during incision of the skin tissue and insertion can be prevented as compared with the prior art needle-shaped member.

In FIG. 1, the shape of the base 1 is a frustum of cone, but may be formed as another shape of revolution (e.g., cylinder), a frustum of triangular pyramid, a frustum of quadrangular pyramid, or a frustum of another polyangular pyramid. The inventors of the present invention has carried out many experiments in order to study suitable values of shape and dimensions of the base 1.

In the FIG. 1, the tip angle θ of the base 1 with a shape of frustum of cone on the side of the blade 2 is set to 45° or less and 5° or more (45°≥θ≥5°).

In a case that the tip angle θ of the base 1 on the side of the blade 2 is more than 45°, the whole base 1 (i.e., the microneedle 10) cannot be inserted into the skin tissue even after the skin tissue is incised (is cut) by the blade 2. This is confirmed by the experiments which have been carried out by the inventors.

On the other hand, in a case that the tip angle θ of the base 1 on the side of the blade 2 is less than 5°, the skin tissue may be incised by the blade 2, but the microneedle may be bent during insertion of the base 1 into the skin tissue. This is also confirmed by the experiments which have been carried out by the inventors.

In the experiments having been carried out by the inventors, the tip angle θ of the base on the side of the blade is suitably 14°>θ>10°.

Next, the length L of the microblade 10 in the central axis direction, the length H of the base 1 in the central axis direction, and the length S of the blade 2 in the central axis direction are not particularly limited, based on the experiments which have been carried out by the inventors. The lengths L, H, and S may be appropriately determined in consideration of variations of a part to be punctured with the microblade and a position, an individual variation, and the like, as long as the tip of the blade 2 can reach at least the dermis and breakage of the blade is not caused (i.e., "bending" of the blade 2 is not caused) during insertion into the skin tissue.

The microneedle 10 is manufactured in manners described below. In an manufacture of the microneedle 10, the material for molding can be selected as long as it is hyaluronic acid, a hair growth promoter, a supernatant of a culture broth in which stem cells are cultured, or another material capable of activating the skin.

Since the material is a liquid phase, during molding, the material for molding is solidified by cooling or removal of moisture. In a manner for removing moisture, for example, the material for molding can be heated to evaporate moisture, or the pressure can be reduced to evaporate moisture.

In the microneedle 10 of the first embodiment, the base 1 is not constructed as a hollow shape, but the base 1 is constructed as a so-called "solid" shape.

Since the microneedle 10 is made from the material capable of activating the skin, the dermis (or the whole skin) can be activated by the microneedle 10 itself when the microneedle 10 reaches the dermis. Therefore, in the microneedle 10, it is not necessary to form a pipe or conduit in the microneedle and it is not necessary to inject the material capable of activating the skin through said pipe, unlike an injection needle.

In a case that the microneedle 10 is made from a metal in a circular pipe shape as like as the injection needle, there is a possible that the metal may be bent during insertion into the skin tissue and remain in the skin tissue. In order to prevent said possibility or risk, a hollow circular pipe shape formed from the metal (injection needle shape) is not adopted in the embodiment shown in the drawings.

However, the base 1 may be formed as a hollow shape.

A particularity of the blade 2 is shown in detail in Figs. 2 to 4.

As shown in FIG. 2, the blade 2 is formed consecutively from the tip side of the base 1 (the upper side in FIG. 2) in an upward direction, and the base 1 and the blade 2 are integrally formed. A tip part of the base 1 has a circular cross section, a stair part 1A (a shoulder part) is formed on an end surface of the tip part on the blade 2 side.

In the experiments by the inventors, a diameter D of the stair part 1A (shoulder part: Refer to Figs. 3 and 4) is suitably more than 10 µm. In a case that the diameter D of the stair part 1A is 10 µm or less, the plate-shaped blade 2 becomes unstable as a supporting member.

In FIG. 3, the thickness of the plate-shaped blade 2 continuing to the tip side of the base 1 (the upper side in FIG. 3) is represented by a character W, and the thickness W is sufficiently smaller than the diameter D of the stair part 1A.

In FIG. 4 which is a partially enlarged side view of the microneedle 10, the plate-shaped blade 2 has a width B which is an approximately constant in the longitudinal direction (the vertical direction in FIG. 4) . In the first embodiment, the width B is set to be larger than the diameter D of the stair part 1A. This is because insertion of the stair part 1A (the tip of the base 1) into the skin tissue is facilitated by longer incision than the diameter D of the stair part 1A by means of the blade 2.

In FIG. 3, the thickness W of the plate-shaped blade 2 is set (is designed) within a range of 20 µm≥W≥0.1 µm.

In the experiments by the inventors, in a case that the thickness W is more than 20 µm, it is confirmed that the skin tissue cannot be incised and the tip cannot be inserted into the skin tissue.

On the other hand, in a case that the thickness W of the blade 2 is less than 0.1 µm, it is confirmed that processing of the blade 2 is difficult (during manufacturing of the mold 20 for the blade 2, a laser beam is not sufficiently focused) .

In the experiments by the inventors, the thickness W of the blade 2 is suitably several micrometers.

The width B of the plate-shaped blade 2 shown by FIG. 4 (the dimension in a direction being orthogonal to the central axis: the dimension in the horizontal direction in FIG. 4) is set within a range of 50 µm≥B≥2 µm.

In the experiments by the inventors, in a case that the width B is more than 50 µm, it is confirmed that the skin tissue cannot be incised and the tip cannot be inserted into the skin tissue.

Also, in a case that the width B is less than 2 µm, it is confirmed that the blade 2 may be broken or bent during incision of the skin tissue and insertion.

Therefore, in the experiments by the inventors, the width B of the blade is suitably within a range of 20 µm≥B≥10 µm.

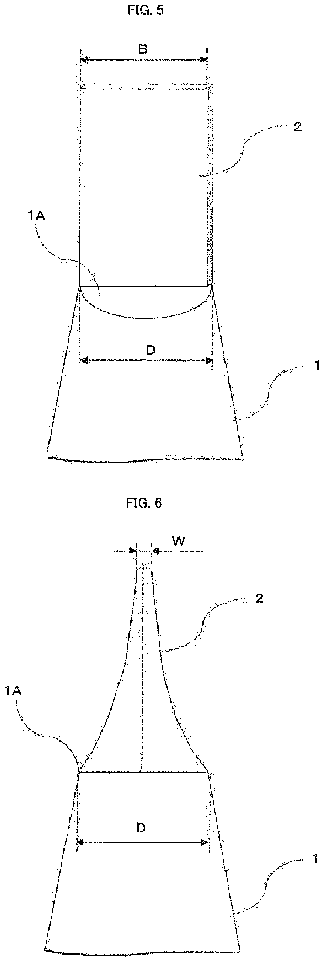

Next, FIG. 5 shows a first modification of the microneedle shown in Figs. 1 to 4.

In FIG. 5, the width B of the plate-shaped blade 2 is set to be substantially equal to the diameter D of the stair part 1A at the blade side end part of the base 1.

Although not shown clearly in FIG. 5, it is possible to set (design) the width B of the blade 2 to be smaller than the diameter D of the stair part 1A (B<D). However, in a case that the width B of the blade 2 is excessively smaller than the diameter D of the stair part 1A, the stair part 1A of the base 1 cannot be inserted into the skin tissue due to interference during incision of the skin tissue by the blade 2. In order to prevent such a problem, it is necessary that the width B of the blade 2 and the diameter D are designed suitably.

Constructions and functional effects other than those described above in the first modification shown in FIG. 5 are the same as those of the microneedle shown in Figs. 1 to 4.

FIG. 6 shows a second modification of the microneedle shown in Figs. 1 to 4.

In FIG. 6, the thickness W of the plate-shaped blade 2 is increased in the axial direction of the microneedle 10 toward the side of the base 1 (the lower side in FIG. 6) and the plate-shaped blade 2 has a smooth curve. The thickness W of the blade 2 at the end part on the base 1 side is equal to the diameter D of the stair part 1A of the base 1.

In the second modification, since there is not a stair part at a boundary between the blade 2 and the base 1, the blade 2 can cut the skin tissue to be incised smoothly and the base 1 (the microneedle 10) can be inserted into the dermis smoothly. Other constructions and functional effects in the second modification of FIG. 6 are the same as those of the microneedle shown in Figs. 1 to 4.

Although not shown in the drawings, in a case that a large number of microneedles 10 are fixed in each of sheet-shaped members and the sheet-shaped members are connected to each other, a sheet, which has a large number of the fixed microneedles 10 and an area of which is large, can be manufactured.

In a case that such a sheet (e.g., an A4-size sheet in which many microneedles 10 are implanted: microneedle sheet) is manufactured, a device being suitable for a shape of use part (e.g., a hair part) of individual user, that is, so-called "made-to-order" device can be provided by appropriate processing of the sheet (microneedle sheet).

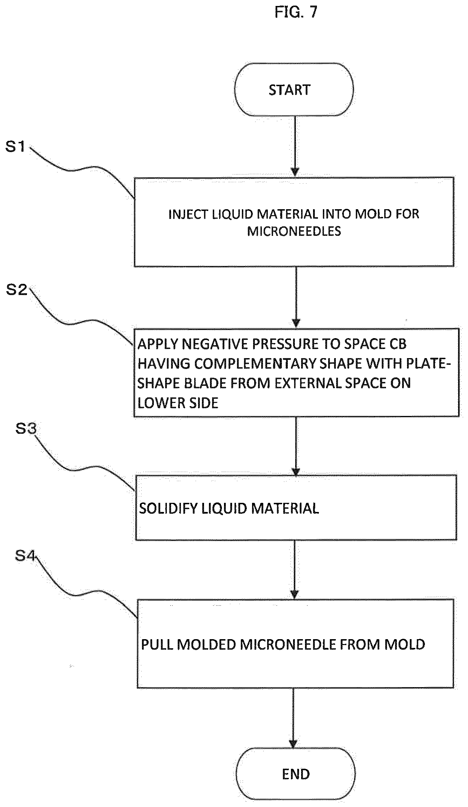

Next, with reference to Figs. 7 to 10, a method for manufacturing the microneedle 10 described with reference to Figs. 1 to 6 will be explained.

First, with reference to FIG. 7, a procedure of manufacturing the microneedle 10 described with reference to Figs. 1 to 4 will be described.

As described below, a mold for manufacture of the microneedle 10 includes a mold 20 (see Figs. 11 to 13) and a mold 30 (see Figs. 14 to 17). The molds 20 and 30 are different depending on a manufacturing method, but the constructions and the functional effects thereof are the almost same. Therefore, in Figs. 7 to 10, a case for manufacture, in which any of the molds 20 and 30 are used, will be described.

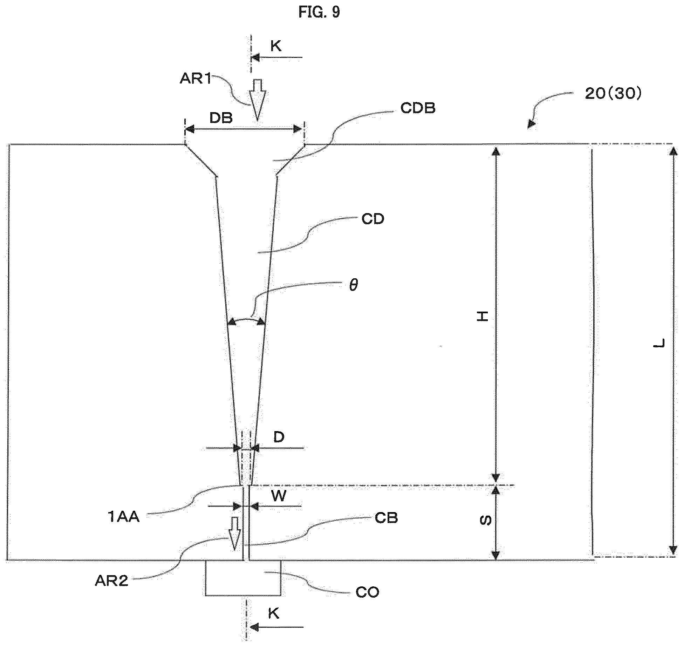

The microneedle 10 is molded using the mold 20 (30) (FIG. 9) for the microneedle 10 in the same manner as an injection molding of a thermoplastic resin.

As described in detail with reference to FIG. 9, the mold 20 (30) for the microneedle 10 has a space CD (FIG. 9) having a complementary shape with the base 1 of the microneedle 10 and a space CB (penetrating groove: FIG. 9) having a complementary shape with the plate-shaped blade 2. The space CB having a complementary shape with the plate-shaped blade 2 communicates with a hollow part CO existing outside the mold 20 (30) (see FIG. 9). In other words, the spaces CD and CB having a complementary shape penetrate the mold 20 (30).

In FIG. 7, Step S1 is a step for supplying a liquid material for manufacture of the microneedle 10, the material for the microneedle 10 (liquid phase material) is injected (see an arrow AR1 in FIG. 9) into the mold 20 (30) of the microneedle 10.

The material for manufacture of the microneedle 10 can be selected from hyaluronic acid (e.g., low molecular weight hyaluronic acid), a hair growth promoter, a supernatant of a culture broth in which stem cells are cultured, and another material capable of activating the skin (the drug or the like), depending on the purpose, as described above.

Herein, the liquid phase material can be solidified by cooling or removal of moisture.

In Step S2, the hollow part CO communicating with the space CB having a complementary shape with the plate-shaped blade 2 (penetrating groove) is decompressed. By means of the decompression of the hollow part CO, a negative pressure is applied to the space CB side of the hollow part CO (penetrating groove) of the mold 20 (30) for the microneedle 10. Herein, the hollow part CO can be decompressed by a publicly known method.

Upon the lower side of the mold 20 (30) (the side of the space CB having a complementary shape with the blade) is communicated with a reduced pressure environment, the material for molding of the microneedle (10) (liquid material) which is supplied in Step S1 is adsorbed due to the negative pressure (an arrow AR2 direction in FIG. 9), and the spaces CB, CD, and CDB in the mold 20 (30) are certainly filled with the material.

After the space CB (penetrating groove) is filled appropriately with the liquid phase material, the hollow part CO is removed from the mold 20 (30).

In next Step S3, the material for molding of the microneedle 10 supplied to the mold 20 (30) (liquid phase material) is solidified.

In solidification of the above-mentioned liquid phase material, a procedure is carried out, in which procedure the material for molding is cooled or in which procedure moisture of the material is removed. In order to remove the moisture, the material for molding may be heated so as to evaporate the moisture or the pressure may be reduced so as to evaporate the moisture.

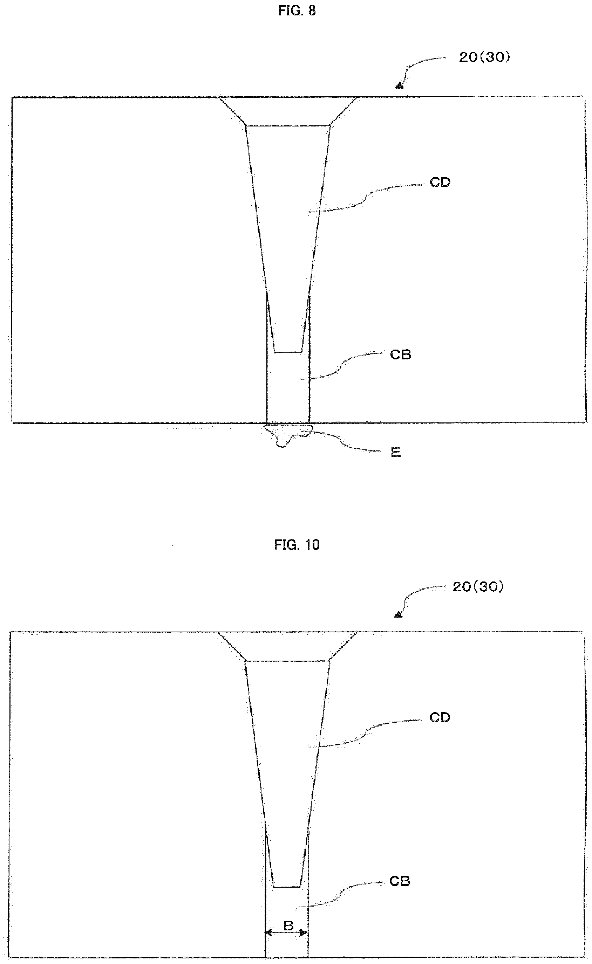

As shown in FIG. 8, a slight amount of the material for molding (liquid phase material) may be discharged from the lower side of the mold 20 (30) from which the hollow part CO is removed, and the discharged material is solidified as a discharged manner thereof. In FIG. 8, the material for molding solidified while it is discharged from the mold 20 (30) is represented by a reference symbol E, and the space having a complementary shape with the base 1 is represented by a reference symbol CD.

In a case shown in FIG. 8, the material E, which is discharged from the mold 20 (30) and solidified, is cut by known means such as a cutter (not shown).

After the material E which is discharged from the mold 20 (30) and solidified is cut, Step S4 in FIG. 7 is carried out.

In Step S4, the molded microneedle 10 is pulled from the mold 20 (30) toward an upper side of the mold 20 (30) (the upper side in Figs. 8 and 9).

Although the mold 20 (30) used in manufacture of the microneedle 10 according to the first embodiment is constructed as an integrated mold, it is possible to construct the mold 20 (30) as a split-type mold by which split-type mold the molded microneedle 10 can be easily pulled from the mold 20 (30).

The mold 20 (30) will be described with reference to FIG. 9.

In FIG. 9, the mold 20 (30) for the microneedle 10 has the space CD having a complementary shape with the base 1 and the space CB having a complementary shape with the plate-shaped blade 2.

The space CDB is a part of the space CD having a complementary shape with the base 1, and the space CDB has a complementary shape with the bottom surface part 1B (FIG. 1) of the base 1. The space CDB constructs a vicinity of end part (in FIG. 9, upper end) of the space CD, and the space CDB is opened upwardly in the mold 20 (30).

The upwardly opened space CDB has a function being for operating as an injection inlet when the mold 20 (30) is filled with the liquid material for manufacture of the microneedle 10.

The end part of the space CD having a complementary shape with base 1 is communicated with the end part (the upper end part in FIG. 9) of the space CB having a complementary shape with the plate-shaped blade 2, and the space CD is a space having a shape of frustum of cone having an angle θ at an end part on the space CB side thereof.

In the space CD having a complementary shape with the base 1, the length in the microneedle central axis direction (the vertical direction in FIG. 9) is represented by a reference symbol H, and said length is equal to the length H of the base 1. The inside diameter D of a stair part 1AA (shoulder part) having a complementary shape with the stair part 1A corresponds to the outside diameter D of the stair part 1A of the microneedle 10. Addition, the maximum inside diameter of the space CDB (in FIG. 9, the inside diameter at the upper end part of the space CDB) is represented by a reference symbol DB.

In the space CB (groove) having a complementary shape with the plate-shaped blade 2, the upper end part is communicated with the space CD, and the lower end part penetrates the mold 20 (30) and is opened downwardly in the mold 20 (30).

In the groove-shaped space (penetrating groove) CB, the length S in the microneedle central axis direction (the vertical direction in FIG. 9) is equal to the length S of the plate-shaped blade 2, the thickness W thereof is equal to the thickness W of the blade 2, and the width B thereof is equal to the width B of the blade 2.

As shown in FIG. 10, the direction of the width B is a dimension extending in a direction perpendicular to the plane of FIG. 9.

During processing of the space CB having a complementary shape with the plate-shaped blade 2 (penetrating groove), for example, the target space CB is irradiated with a laser beam (see Figs. 11 to 13). Alternatively, a layer coated with a photoresist is irradiated with light to perform processing (see Figs. 14 to 17).

Also, upon the mold 20 (30) is filled with the molding material for manufacture of the microneedle 10 as mentioned above, the hollow part CO communicating with the space (CB) (penetrating groove) is decompressed by a well-known decompression device (not shown) to change the side of the space (CB) (penetrating groove) into a reduced pressure environment. Thus, the molding material injected into the mold 20 (30) is adsorbed due to the negative pressure (the arrow AR2 direction in FIG. 9), and the spaces CB and CD in the mold 20 (30) are certainly filled with the molding material.

By communicating the space CB (penetrating groove) with the reduced pressure environment, a case that the space CB is not filled to the tip thereof with the material for molding (liquid phase material) due to the surface tension can be prevented.

Further, although gas is generated during filling the mold 20 (30) with the material for molding, the material for molding (liquid phase material) is certainly inserted into the spaces CD and CB by communicating the space CB (penetrating groove) with the reduced pressure environment.

Herein, in a case that the degree of vacuum is too high during communicating the space CB (penetrating groove) with the reduced pressure environment, it is possible that the liquid material for molding injected into the mold 20 (30) may be boiled. Therefore, decompression is controlled within such a range that the liquid material will not be boiled.

Although only a space corresponding to one microneedle 10 is formed in the mold 20 (30) shown in FIG. 9, it is possible to form a plurality of spaces CB and CD having a complementary shape with a plurality of microneedles 10. In a case that a mold being capable of manufacturing a plurality of microneedles 10 (not shown) is used, the plurality of microneedles 10 can be molded simultaneously.

A sheet-shaped member in which a large number of microneedles 10 are implanted is easily molded. As a result, a device suitable for a shape of use part (e.g., a hair part) of individual user (so-called "made-to-order" device) can be manufactured by means of said sheet-shaped member.

The method for manufacturing the mold 20 of the microneedle 10 will be described with reference to Figs. 11 to 13.

FIG. 11 shows a material 21 for the mold 20 before processing (workpiece). The workpiece 21 has a solid rectangular parallelepiped shape. In consideration of size (length L, width B, and the like) of the microneedle 10 to be molded, a number of microneedle 10 to be molded simultaneously by means of one mold 20, and the like, the size of the workpiece 21 is determined.

A material for the workpiece 21 can be selected, for example, from glass, alumina, various types of resins, nickel, and nickel-cobalt-based alloy.

FIG. 12 shows a step of processing a region CD having a complementary shape with the base 1 and a region CDB having a complementary shape with the bottom surface part 1B (see FIG. 1) of the base 1.

In FIG. 12, the workpiece 21 is set to a publicly known cutting device (not shown), the space CD having a complementary shape with the base 1 is cut, and the space CDB having a complementary shape with the bottom surface part 1B of the base 1 is cut. A reference symbol 1A represents a stair part between the base 1 and the blade 2.

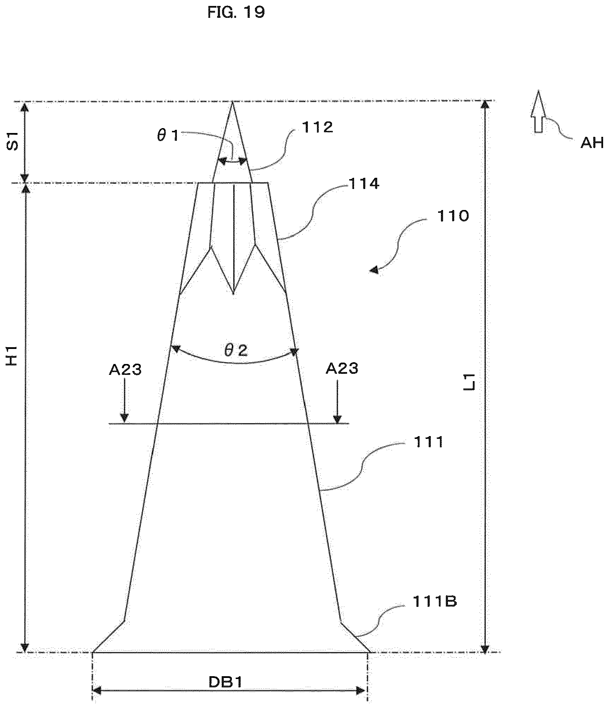

In the first embodiment, the shape of the base 1 of the microneedle 10 is a frustum of cone, and the space CD has a complementary shape which shape is a frustum of cone. However, in a case that the shape of the base 1 is a shape of revolution other than a frustum of cone (e.g., cylinder: see FIG. 19), a frustum of triangular pyramid, a frustum of quadrangular pyramid, or a frustum of another polyangular pyramid, a space having a complementary shape with the shape of the base 1 is processed by a known processing method.

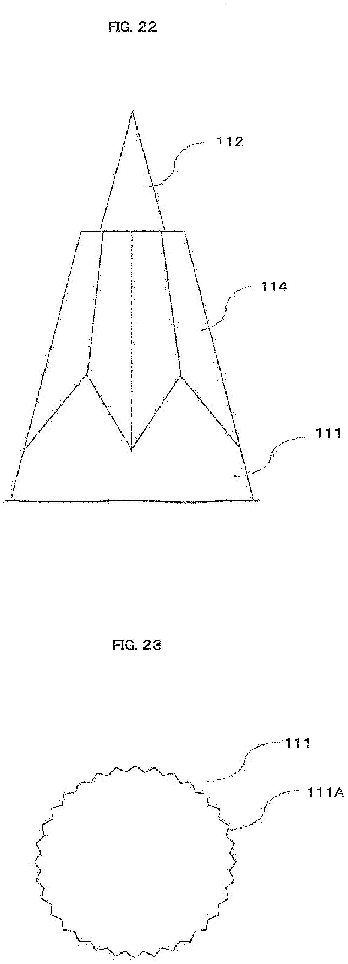

Further, in addition to a smooth surface shape, a shape in which irregularities are continuously formed in a circumferential direction can be selected as a cross-sectional shape of the base 1 (see FIG. 23). Such the shape can be easily processed, for example, by manufacturing molds 20, 21, 30, and 31 described below by laser processing.

As described above, the bottom surface part 1B of the base 1 can be omitted. In this case, processing of the space CDB is omitted.

FIG. 13 shows a step in which the space CB having a complementary shape with the plate-shaped blade 2 (penetrating groove) is processed, after processing of the regions CD and CDB.

In FIG. 13, after the spaces CB and CDB having a shape of frustum of cone are formed, the workpiece 21 is irradiated from the bottom (from the lower side in FIG. 13) with a laser beam, so as to process (cut) a groove-shaped space CB (penetrating groove, a hatched part).

In FIG. 13, an arrow R represents an irradiation direction of the laser beam.

In FIG. 13 which is shown in a direction of arrows K-K in FIG. 9, a movement direction is represented by an arrow F, in which direction the laser beam R is moved by the width B of the blade 2 in the width direction of the blade 2 (an arrow F direction in FIG. 13) during processing by the laser beam R.

At that time, the workpiece 21 is irradiated with the laser beam R so that a plate-shaped space CB processed by the laser beam R (penetrating groove) divides the space CD into two parts in a direction perpendicular to the paper of FIG. 13. As a result, the space CB (penetrating groove) as shown in FIG. 10 is formed in the workpiece 21.

By carrying out the steps mainly described with reference to Figs. 11 to 13, the mold 20 shown in FIG. 9 is manufactured. The base 1 and the plate-shaped blade 2 of the microneedle 10 manufactured using the mold 20 are integrally formed and will not be separated.

It is to be noted that, if the space CB (a space or region having a complementary shape with the blade 2) having a thickness W (20 µm≥W≥ 0.1 µm) can be processed, a method other than the laser beam can be used in the step for processing of the space CB (penetrating groove).

Next, the method for manufacturing the mold 30 will be described with reference to Figs. 14 to 17.

FIG. 14 shows a material 31 (workpiece) for the mold 30 before processing.

The workpiece 31 has a workpiece body part 31A which is made from a material having a property of transmitting light (e.g., glass), and a photoresist part 31B which is made from a known photoresist and is layered on one surface (the top surface in FIG. 14) of the workpiece body part 31A. Herein, the space CB having a complementary shape with the plate-shaped blade 2 (penetrating groove) is processed in the photoresist part 31B, and the spaces CD and CDB having a complementary shape with the base 1 are processed in the workpiece body part 31A.

A "negative" photoresist is used as the photoresist.

The workpiece 31 (the workpiece body part 31A and the photoresist part 31B) has a solid rectangular parallelepiped shape. In consideration of size of the microneedle 10 to be molded, the number of microneedle 10 to be molded simultaneously using one mold 30, and the like, the size of the workpiece 31 is determined.

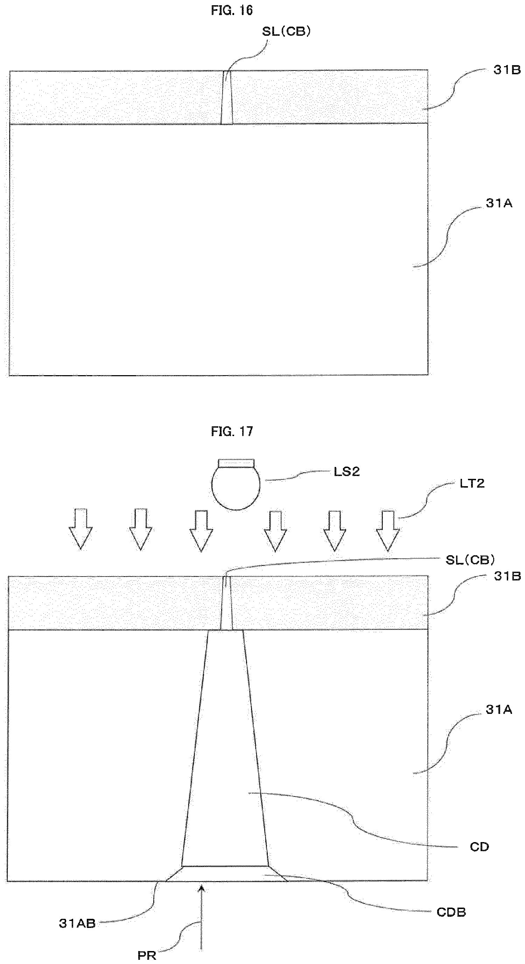

With reference to Figs. 15 and 16, a step of processing the space CB having a complementary shape with the plate-shaped blade 2 will be described.

In the step shown in FIG. 15, a small shading member G is disposed on an upper surface of the photoresist part 31B of the workpiece 31, and the photoresist part 31B, on which the shading member G is disposed, is irradiated with light (arrows LT1) from a light source LS1 which positioned outside of the workpiece 31.

Herein, a cross-sectional shape of the shading member G which is irradiated with light corresponds to a cross-sectional shape of the space CB having a complementary shape with the plate-shaped blade 2.

Since the "negative" photoresist is cured by irradiation with light, a region being other than an area directly below the shading member G in the photoresist part 31B is cured by irradiation with light, and therefore, the strength of said region becomes necessary strength for manufacturing a mold.

On the other hand, the area directly below the shading member G in the photoresist part 31B is shaded, and therefore, the region is not cured by irradiation with light. For this reason, an uncured photoresist at the area directly below the shading member G is easily removed by post-processing (e.g., washing procedure).

FIG. 16 shows a state in which the uncured photoresist at the area directly below the shading member G is removed and a slit SL is formed in the photoresist part 31B. The shape of the slit SL corresponds to the shape of the space CB having a complementary shape with the plate-shaped blade 2 (FIG. 9: penetrating groove). Various dimensions of the slit SL are designed so as to correspond to the length S in the central axis direction or the like of the space CB shown in FIG. 9.

Herein, in the "negative" photoresist, since an area of the region being cured by irradiation with light is decreased as the region is farther from the light source (in Figs. 15 and 16, as the region is disposed on a lower side), the slit SL at the region which is not cured by irradiation with light has a shape an area of which is increased as toward the lower side as shown in FIG. 16. Such a shape is convenient for pulling of the microneedle 10 from the mold after solidification.

On the other hand, in a case that a "positive" photoresist is used, since an area of the region which is not cured by irradiation with light is decreased as the region is farther from the light source (in Figs. 15 and 16, as the region is disposed on the lower side), the formed slit has a shape an area of which is decreased toward the lower side in FIG. 16 and such the shape of the slit is not convenient for pulling from the mold.

It is to be noted that an upper end of the slit SL (space CB) is opened outside the workpiece 31 (photoresist part 31B) .

After the slit SL (space CB) is formed, in the step shown in FIG. 17, the workpiece body part 31A is processed into the spaces CD and CDB having a complementary shape with the base 1 of the microneedle 10.

In FIG. 31, in a case that the workpiece 31 is irradiated with light of the light source LS2 from the side in which the space CB (penetrating groove) is formed (photoresist part 31B side; upper side in FIG. 17), a position of the slit SL (space CB) can be confirmed from a bottom surface 31AB side of the workpiece body part 31Amade from the material transmitting light (e.g., glass) . In a situation that the position of the slit SL can be confirmed, the spaces CD and CDB having a complementary shape with the base 1 and communicating with the lower end part of the space CB (penetrating groove) are cut (processed) by irradiation with a known laser beam (e.g., pulse laser: arrow PR) from the bottom surface 31AB of the workpiece body part 31A.

Thus, the mold 30 having the spaces CD, CDB, and CB having a complementary shape with the microneedle 10 (the base 1 and the plate-shaped blade 2) is completed. The base 1 and the plate-shaped blade 2 of the microneedle 10 manufactured by means of the mold 30 are integrally formed, and therefore, The base 1 and the plate-shaped blade 2 are not separated.

It is to be noted that, instead of the pulse laser, known means being capable of precision work a degree of which is as same as the pulse laser's precision work.

In the method for manufacturing the mold 30 shown in Figs. 14 to 17, so-called "dry etching" and "wet etching" are used during formation of the space CB having a complementary shape with the blade 2 size of which is fine. In a case that the laser beam cannot be not converged (focused) to a degree in which the space CB having a complementary shape with the blade 2 can be cut, the mold 30 having the space CB having a complementary shape with the blade 2 with very fine size can be manufactured.

Other constructions and functional effects in the manufacturing method of Figs. 14 to 17 are the same as those in the manufacturing method shown in Figs. 11 to 13.

In the microneedle 10 according to the first embodiment, since the plate-shaped blade 2 is integrally formed at the end part of the base 1 and the thickness W of the blade 2 is set (designed) to a fine dimension (20 µm≥W≥0.1 µm), the blade 2 has a sharp shape and can cut (incise) the skin tissue to be incised. When the skin tissue is incised by the blade 2, the base 1 integrally molded with the blade 2 can also easily be inserted into the skin tissue.

Accordingly, the microneedle 10 made from the material capable of activating the skin (hyaluronic acid, a hair growth promoter, a supernatant of a culture broth in which stem cells are cultured, or the like) can be inserted into the dermis T, the drug or the like which is the material constructing the microneedle 10 can be supplied to the dermis T, and then, the skin can be activated.

Since the microneedle 10 according to the first embodiment has the plate-shaped blade 2, the microneedle 10 can be easily manufactured by means of the mold 20 or 30. Therefore, the tip shape is not formed into a spherical shape or a curved shape, the blade 2 can cut (incise) the skin tissue to be certainly incised and the microneedle 10 can be inserted into the dermis T.

In a case that the dimensions (thickness W, width B, length S, and Figs. 3 and 4) and the strength of the plate-shaped blade 2 are appropriately designed, bending can be prevented during incision of the skin tissue and insertion, as compared with the needle-shaped member.

According to the method for manufacturing the microneedle 10 according to the first embodiment, since the penetrating groove below the region having a complementary shape with the base 2 in the used mold 20 or 30 is formed (on the side of the region CB having a complementary shape with the plate-shaped blade 2) and the step for communicating the lower side of the mold 20 or 30 (the side of the region CB having a complementary shape with the plate-shaped blade) with the reduced pressure environment is carried out, the material for molding of the microneedle 10 (liquid material) is adsorbed due to the negative pressure, and the space (regions CB, CD, and CDB) in the mold 20 or 30 is certainly filled with the material.

Accordingly, it is possible to prevent a case that the mold 20 or 30 is not filled to the tip (whole region CB) thereof with the material for molding due to surface tension, the mold 20 or 30 is filled to the tip thereof (the whole region CB) with the material for molding although gas is generated.

Further, since the microneedle 10 according to the first embodiment is manufactured by means of the mold 20 or 30, it is easy to manufacture a sheet-shaped member in which many microneedles 10 are fixed.

Then, the sheet-shaped member is connected to another sheet-shaped member so as to manufacture a sheet in which many microneedles 10 are fixed and an area of which is large. In a case that such a sheet having a large area is appropriately processed, a device suitable for a shape of use part (e.g., a hair part) of individual user can be provided as a so-called "made-to-order" device.

With reference to Figs. 19 to 24, a second embodiment of the present invention will be described.

In FIG. 19, a microneedle according to the second embodiment is entirely represented by a reference symbol 110. The microneedle 110 has a conical tip part 112 on a tip side (arrow AH side: upper side in FIG. 19) and a base 111 having a shape of frustum of cone (a region on an opposite side to the arrow AH of the conical tip part 112: the lower region in FIG. 19) . A part having a star polygonal cross section 114 is formed in a region between the base 111 and the conical tip part 112. The conical tip part 112, the base 111 and the part having a star polygonal cross section 114 are integrally formed.

The cross-sectional shape of the part having a star polygonal cross section 114 is a star hexagon. However, the cross-sectional shape may be a star pentagon or another star polygonal.

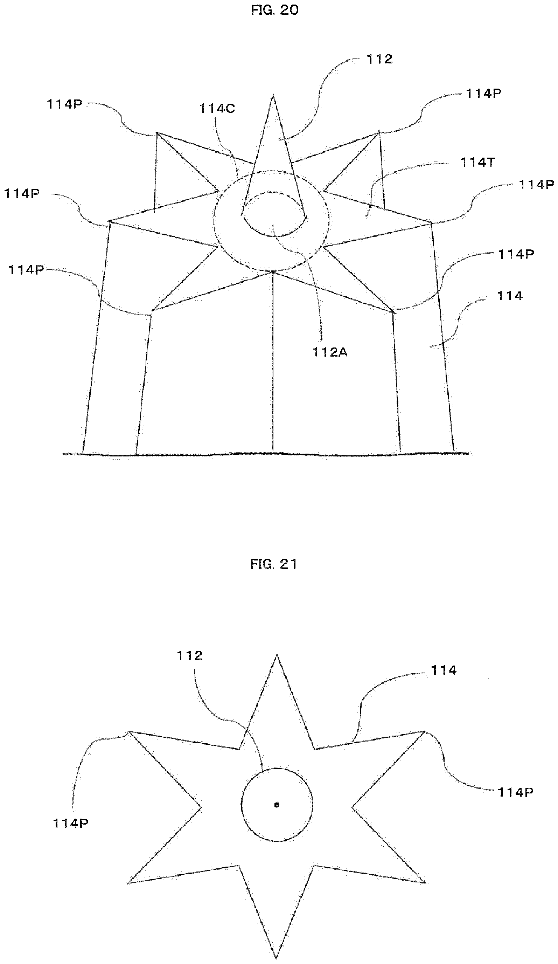

As shown in Figs. 19, 20, and 21, the part having a star polygonal cross section 114 having six tips 114P (ridges) has a top part 114T (FIG. 20) of star hexagonal cross section adjacent to the conical tip part 112. In the inside area of a basic circle 114C (virtual circle represented by a dotted line in FIG. 20) of the star hexagon in the top part 114T, a bottom part 112A of the adjacent conical tip part 112 is housed. It is to be noted that the basic circle 114C may not be an exact circle.

As described above, the cross-sectional shape of the part having a star polygonal cross section 114 is a star hexagon (FIG. 21) . As shown in FIG. 23, the cross-sectional shape of the base 111 is substantially a circle and the peripheral portion thereof has a shape in which irregularities 111A (concavity and convexity) are continuously formed. As shown in Figs. 19 and 22, although a stair part (top part 114T) is formed in a boundary between the part having the star polygonal cross section 114 and the base 111, the star polygonal cross section 114 and the base 111 are integrally connected.

As shown in FIG. 19, the diameter of a part in the vicinity of the bottom surface (bottom surface part 111B) of the base 111 is abruptly increased. In FIG. 19, the maximum diameter (diameter of the lower end part in FIG. 19) of the bottom surface part 111B is represented by a reference symbol DB1.

In a case that a large number of microneedles 110 are attached to one sheet (assembly of sheet-shaped member, not shown), each manufactured microneedle 10 is bonded to the sheet-shaped member coated with an adhesive (not shown) and stored, the bottom surface part 111B is certainly bonded to the sheet-shaped member due to the large diameter of the bottom surface part.

It is to be noted that the base bottom surface part 111B in which the diameter is abruptly increased may be omitted.

In FIG. 19, the tip angle θ1 of the conical tip part 112 is designed (set) to 14° or less (14°≥θ1). In a case that the tip angle θ1 of the conical tip part 112 is larger than 14°, the skin tissue cannot be incised (cut) and the tip cannot be inserted into the skin tissue. Such the fact is confirmed by the experiments being carried out by the inventors.

The tip angle θ2 of the part having a star polygonal cross section 114 and the base 111 on the side of the conical tip part 112 is also set to 14° or less (14°≥θ2). In a case that the tip angle θ2 of the part having a star polygonal cross section 114 and the base 111 on the side of the conical tip part 112 is larger than 14°, it is difficult that the part having a star polygonal cross section 114 and the base 111 (i.e., the microneedle 110) are inserted into the skin tissue after the skin tissue is incised by the conical tip part 112. Such the fact is confirmed by the experiments being carried out by the inventors.

In FIG. 19, the length of the microneedle 110 in a central axis direction is represented by a reference symbol L1, the length of the base 111 and the part having a star polygonal cross section 114 in the central axis direction is represented by a reference symbol H1, and the length of the conical tip part 112 in the central axis direction is represented by a reference symbol S1.

The lengths L1, H1, and S1 are appropriately determined in consideration of variations of a part to be punctured with the conical tip part 112 and a position, an individual variation, and the like, as long as the conical tip part 112 can reach at least the dermis and the microneedle 110 is not broken (i.e., "bending" is not caused) during insertion into the skin tissue.

A material for molding the microneedle 110 in the second embodiment can be adopted as long as it is hyaluronic acid, (e.g., low molecular weight hyaluronic acid), a hair growth promoter, a supernatant of a culture broth in which stem cells are cultured, or another material capable of activating the skin, as like the first embodiment. During molding, a liquid phase material for molding is solidified by cooling or removal of moisture.

Also, in the microneedle 110 of the second embodiment, the conical tip part 112, the base 1, and the part having a star polygonal cross section 114 do not have a hollow shape (a shape having a cavity part inside thereof), and have a so-called "solid" shape (a shape having no cavity part inside thereof). However, it is possible that a part or all of the conical tip part 112, the base 111 and the part having a star polygonal cross section 114 which construct the microneedle 110 have a hollow shape.

In the second embodiment of Figs. 19 to 23, the part having a star polygonal cross section 114 is formed in a region between the base 111 and the conical tip part 112 of the microneedle 110. When the skin is punctured with the microneedle 110, the skin tissue is incised (cut) by the conical tip part 112, and then the skin is incised by the tips 114P (ridges) of the part having a star polygonal cross section 114 following to the conical tip part 112.

Therefore, the part having a star polygonal cross section 114 and the base 111 of the microneedle 110 are easily inserted into the skin tissue, and the microneedle 110 is easily inserted into the dermis T.

Other constructions and functional effects and matters with respect to manufacturing the second embodiment shown in Figs. 19 to 23 are the same as those in the first embodiment shown in Figs. 1 to 18.

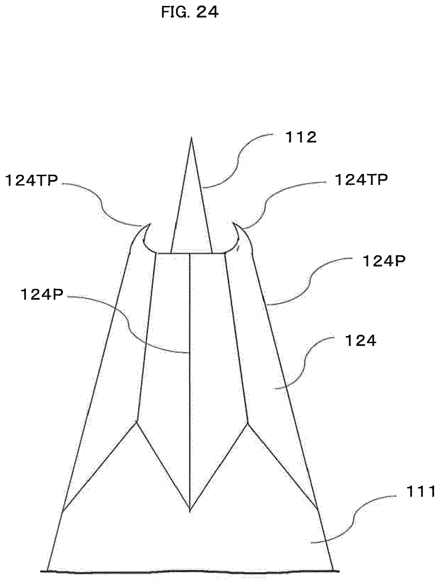

FIG. 24 shows a modification of the second embodiment. In the modification shown in FIG. 24, a tip part of a part having a star polygonal cross section 124 on the conical tip part 112 (in FIG. 24, an upper end part of the part having a star polygonal cross section 124) forms a sharp top part 124TP protruding on the side of the conical tip part 112 (on the upper side in FIG. 24) .

Since an end part of the part having a star polygonal cross section 124 (in FIG. 24, the upper end part of the part having a star polygonal cross section 124) forms the sharp top part 124TP protruding on the side of the conical tip part 112 (on the upper side in FIG. 24), when the skin is punctured with the microneedle 110, the skin tissue is also incised (cut) by the sharp top part 124TP. As a result, not only tips 124P (ridges) of the part having a star hexagonal cross section 124 but also the sharp top part 124TP can incise (cut) the skin tissue to be incised, and therefore, the part having a star hexagonal cross section 124 and the base 111 are further easily inserted into the skin tissue.

Other constructions and functional effects and matters with respect to manufacture in the modification shown in FIG. 24 are the same as those in the second embodiment shown in Figs. 19 to 23.

The shown embodiments are merely illustrative, and are not intended to limit the technical scope of the present invention.

For example, the "drug or the like" as the material for the microneedle of the present invention is a drug, a beauty article, or a substance which can be contributed simply and certainly to the activity of the skin in the skin surface layer, and widely includes hyaluronic acid (e.g., low molecular weight hyaluronic acid), a hair growth promoter, a supernatant of a culture broth in which stem cells are cultured, another material capable of activating the skin, insulin, an anticancer drug, a hair growth promoter, a therapeutic drug for an auditory disorder, a therapeutic drug for allergy (e.g., "allergy to pollen"), a therapeutic drug for empyema, a drug for Alzheimer's disease and other dementia, a drug for a brain disease, a therapeutic drug for laryngitis, a therapeutic drug for a periodontal disease, a drug for regeneration of alveolar bone, a drug for improvement of oral cavity immunity, a therapeutic drug for prevention of sunburn and inflammation caused by sunburn, a therapeutic drug for psoriasis, a therapeutic drug for atopic dermatitis, a therapeutic drug for decubitus (bedsore), a therapeutic drug for other various skin diseases, a drug for regeneration of skin to remove stretch marks, a drug for regeneration of skin after wound and another surgery, a therapeutic drug for a disease caused by Trichophyton, a drug for prevention of an increase in puncture and ED, and other various drugs (e.g., oriental drug or Chinese drug), although it is not described clearly in the embodiments shown in the drawings.

Further, in the embodiments shown in the drawings, although a case of application to human is mainly described, the present invention can be applied to animals that are likely to suffer a skin disease and are necessary for procedure using the microneedle (dogs, cats, domestic animals such as horses and cattle, camels, and the other animals). In this case, as said "drug or the like" as the material for manufacture of microneedles, a drug suitable for dogs, cats, domestic animals such as horses and cattle, camels, and the other animals (animals that are likely to suffer a skin disease and are necessary for procedure using the microneedle) is used.

### DESCRIPTION OF THE REFERENCE NUMERALS

1, 111: base
1A: stair part (shoulder part)
1B, 111B: bottom surface part
2: plate-shaped blade
10, 110: microneedle
20, 30: mold for microneedles
21, 31: workpiece for mold
31A: workpiece body part
31B: photoresist part
112: conical tip part
114, 124: part having a star hexagonal cross section
CB: region or space having a complementary shape with plate-shaped blade (penetrating groove)
CD: region or space having a complementary shape with base
CDB: complementary region or space with bottom surface part of base
CO: hollow part
G: shading member
LS1, LS2: light source
U: epidermis
Uh: stratum corneum
T: dermis

## Claims

1. A microneedle comprising a base and a plate-shaped blade, wherein the base and the plate-shaped blade are integrally formed and the plate-shaped blade extends from an end part of the base.

2. A method for manufacturing a microneedle comprising steps:
for filling a mold with a liquid material for manufacture of microneedles, said mold having a space having a complementary shape with a base of a microneedle and a space having a complementary shape with a plate-shaped blade, the space having a complementary shape with the plate-shaped blade communicating with an external space; and
for decompressing the space communicating with the space having a complementary shape.

3. A mold used in manufacture of a microneedle comprising a space having a complementary shape with a base of a microneedle and a space having a complementary shape with a plate-shaped blade, wherein the space having a complementary shape with the plate-shaped blade is communicated with an external space.

4. A method for manufacturing a mold used in manufacture of a microneedle comprising steps:
for processing a complementary region with a base of a microneedle; and
for cutting the mold so as to form the space having a complementary shape with the plate-shaped blade by irradiating a laser beam from the bottom of the mold so that the laser beam penetrates to the complementary region with the base of the microneedle and moving the laser beam by a width of the blade in a horizontal direction.

5. A method for manufacturing a mold used in manufacture of a microneedle comprising steps:
for using a workpiece being constructed by a workpiece body part made of a permeable material and a photoresist part formed from a photoresist;
for placing a shading member having the same size as that of a tip surface of the plate-shaped blade of the microneedle on the photoresist part and irradiating with light to the photoresist part from a side on which the shading member is placed;
for forming a space having a complementary shape with the blade by removing the photoresist directly below said shading member; and
for forming a space having a complementary shape with the base by irradiating with light to the workpiece body from a side of the photoresist part and by processing the workpiece body from a side opposite to the light irradiation side.

6. A microneedle comprising a base and a conical tip part, wherein in a region between the base and the conical tip part, a part having a star polygonal cross section is formed and the base, the conical tip part, and the part having a star polygonal cross section are integrally formed.
